(19) Europäisches Patentamt
European Patent Office
Office européen des brevets

(11) **EP 3 199 113 A1**

(12) **EUROPEAN PATENT APPLICATION**
published in accordance with Art. 153(4) EPC

(43) Date of publication:
**02.08.2017 Bulletin 2017/31**

(21) Application number: **16830276.8**

(22) Date of filing: **08.07.2016**

(51) Int Cl.:
**A61B 17/32** (2006.01)          **A61B 17/56** (2006.01)
**A61B 18/12** (2006.01)

(86) International application number:
**PCT/JP2016/070307**

(87) International publication number:
**WO 2017/018171 (02.02.2017 Gazette 2017/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **27.07.2015 JP 2015147875**

(71) Applicant: **Olympus Corporation
Hachioji-shi, Tokyo 192-8507 (JP)**

(72) Inventors:
• **TANIGAMI, Yasuo**
  **Hachioji-shi**
  **Tokyo 192-8507 (JP)**
• **NAKAMURA, Hiroto**
  **Hachioji-shi**
  **Tokyo 192-8507 (JP)**
• **MIYASAKA, Ryo**
  **Hachioji-shi**
  **Tokyo 192-8507 (JP)**

(74) Representative: **Gunzelmann, Rainer
Wuesthoff & Wuesthoff
Patentanwälte PartG mbB
Schweigerstraße 2
81541 München (DE)**

(54) **ENERGY TREATMENT SYSTEM AND ENERGY CONTROL DEVICE**

(57)     In An energy treatment system, a judgment section judges whether a vicinity of a treated target, with which an end effector is in contact, is filled with a liquid, in a state that the end effector is in contact with the treated target and a driving current is being output from an output section. In the energy treatment system, a controller executes at least one of stopping an output of the driving current from the output section and issuing an alert to an effect that the liquid is not filled, based on a fact that the judgment section judged that the vicinity of the treated target is not filled with the liquid.

FIG. 2

**Description**

Technical Field

The present invention relates to an energy treatment system including a treatment instrument configured to treat a treated target in a joint cavity by using energy, and an energy control device which controls the supply of a driving current to the treatment instrument in the energy treatment system.

Background Art

PCT International Publication No. 2010/087060 discloses an energy treatment system in which an end effector treats a treated target in a joint cavity by using energy. In this energy treatment system, electric energy is supplied to a treatment instrument from an energy control device. Thereby, the treatment instrument is driven, and a treatment using energy by the end effector is performed. In addition, in the energy treatment system, in the state in which the treatment using energy is being performed by the end effector, the supply of liquid into the joint cavity and the discharge of liquid from the joint cavity are controlled by a perfusion control device. In addition, in the state in which the end effector is immersed in a liquid (in the state in which a contact part of the end effector, which is in contact with the treated target, is located in a liquid), the end effector performs a treatment by using energy.

When the treatment disclosed in PCT International Publication No. 2010/087060 is being performed, there is a case in which a sufficient amount of liquid is not supplied to the vicinity of the treated target, since the liquid in a liquid supply source is emptied, or the treated target is located in a very narrow space in the joint cavity. In this case, the treated target is treated in the state in which the vicinity of the treated target is not filled with liquid (in the state in which the contact part of the end effector, which is in contact with the treated target, is not located in the liquid). If the treatment using energy is performed in the state in which the vicinity of the treated target is not filled with liquid, the heat produced in the vicinity of the treated target increases, and the performance of treatment deteriorates.

Summary of Invention

The present invention has been made in consideration of the above problem, and the object of the invention is to provide an energy treatment system which effectively prevents a treatment from being performed in a state in which the vicinity of a treated target is not filled with liquid, and an energy control device which is provided in this energy treatment system.

To solve the above mentioned problems, according to one aspect of the invention, an energy treatment system including: a treatment instrument including an end effector configured to treat, in a state in which the end effector is in contact with a treated target in a joint cavity, the treated target by using energy; an output section configured to output to the treatment instrument a driving current which drives the treatment instrument; a judgment section configured to judge whether a vicinity of the treated target, with which the end effector is in contact, is filled with a liquid, in a state in which the end effector is in contact with the treated target and the driving current is being output from the output section; and a controller configured to execute at least one of stopping the output of the driving current from the output section and issuing an alert to an effect that the liquid is not filled, based on a fact that the judgment section judged that the vicinity of the treated target is not filled with the liquid.

According to one another aspect of the invention, an energy control device which is used together with a treatment instrument including an end effector configured to treat, in a state in which the end effector is in contact with a treated target in a joint cavity, the treated target by using energy, the energy control device being configured to control supply to the treatment instrument of a driving current which drives the treatment instrument, the energy control device including: an output section configured to output the driving current to the treatment instrument; a judgment section configured to judge whether a vicinity of the treated target, with which the end effector is in contact, is filled with a liquid, in a state in which the end effector is in contact with the treated target and the driving current is being output from the output unit; and a controller configured to execute at least one of stopping the output of the driving current from the output section and issuing an alert to an effect that the liquid is not filled, based on a fact that the judgment section judged that the vicinity of the treated target is not filled with the liquid.

Brief Description of Drawings

FIG. 1 is a schematic view illustrating an energy treatment system according to a first embodiment of the present invention,

FIG. 2 is a schematic view illustrating the configuration of a treatment instrument and an energy control device according to the first embodiment,

FIG. 3 is a schematic view for describing a process of calculating a phase difference between a driving current and a driving voltage, the process being executed by a calculator according to the first embodiment,

FIG. 4 is a flowchart illustrating a process in the energy control device at a time of performing a treatment by using the energy treatment system according to the first embodiment,

FIG. 5 is a flowchart illustrating a judgment process of the presence or absence of liquid in the vicinity of a treated target, the process being executed by the energy control device according to the first embodiment,

FIG. 6 is a schematic view illustrating an example of a variation with time of an acoustic impedance,

FIG. 7 is a schematic view illustrating an example of variations with time of a driving voltage and a driving current in a state in which the end effector located in a liquid is abrading a treated target, and in a state in which the end effector located in the air is abrading the treated target,

FIG. 8 is a schematic view illustrating measurement data of a voltage value of a driving voltage, a phase difference and an acoustic impedance in a state in which the end effector located in a liquid is abrading a treated target, and in a state in which the end effector located in the air is abrading the treated target,

FIG. 9 is a flowchart illustrating a judgment process of the presence or absence of liquid in the vicinity of a treated target, the judgment process being executed by an energy control device according to a first modification of the first embodiment,

FIG. 10 is a flowchart illustrating a judgment process of the presence or absence of liquid in the vicinity of a treated target, the judgment process being executed by an energy control device according to a second modification of the first embodiment,

FIG. 11 is a schematic view illustrating an example of a variation with time of an integration value of a driving voltage in a state in which the end effector located in a liquid is abrading a treated target, and in a state in which the end effector located in the air is abrading the treated target,

FIG. 12 is a schematic view illustrating the configuration of a treatment instrument and an energy control device according to a second embodiment,

FIG. 13 is a flowchart illustrating a judgment process of the presence or absence of liquid in the vicinity of a treated target, the judgment process being executed by the energy control device according to the second embodiment, and

FIG. 14 is a schematic view illustrating an example of a variation with time of a high-frequency impedance. Description of Embodiments

(First Embodiment)

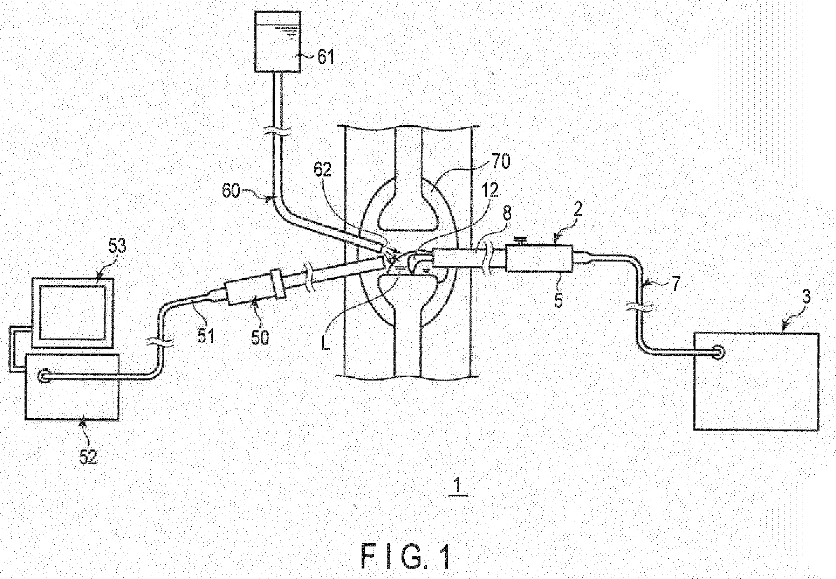

A first embodiment of the present invention will be described with reference to FIG. 1 to FIG. 8. FIG. 1 is a view illustrating an energy treatment system 1. As illustrated in FIG. 1, the energy treatment system 1 includes a treatment instrument (energy treatment instrument) 2, and an energy control device 3 which controls the supply of energy to the treatment instrument 2. The supply to the treatment instrument 2 of a driving current I, which drives the treatment instrument 2, is controlled by the energy control device 3. The treatment instrument 2 includes a housing 5 which can be held. One end of a cable 7 is connected to the housing 5. The other end of the cable 7 is connected to the energy control device 3.

In addition, in the present embodiment, an endoscope (rigid endoscope) 50 and a liquid supply assisting instrument 60 are used together with the treatment instrument 2 and energy control device 3. One end of a universal cord 51 is connected to the endoscope 50, and the other end of the universal cord 51 is connected to an image processing device 52 such as an image processor. Besides, the image processing device 52 is electrically connected to a display device 53 such as a monitor. A subject is imaged by an imaging element (not shown) of the endoscope 50, and thereby an imaging signal is transmitted to the image processing device 52 via an imaging cable (not shown) which extends in the inside of the endoscope 50 and in the inside of the universal cord 51. Thereby, image processing is executed by the image processing device 52, and an image of the subject, which was generated by the image processing, is displayed on the display device 53.

In addition, a liquid supply channel (not shown) extends in the inside of the liquid supply assisting instrument 60, and one end of the liquid supply channel is connected to a liquid supply source 61 such as a liquid bag. Further, the supply of liquid from the liquid supply source 61 through the liquid supply channel is adjusted by an operation by a channel opening or closing member (not shown) such as a clamp (Klemme). A liquid, which is supplied through the liquid supply channel, is jetted out to the outside of the liquid supply assisting instrument 60 from a jet port 62 which is provided at the other end of the liquid supply channel.

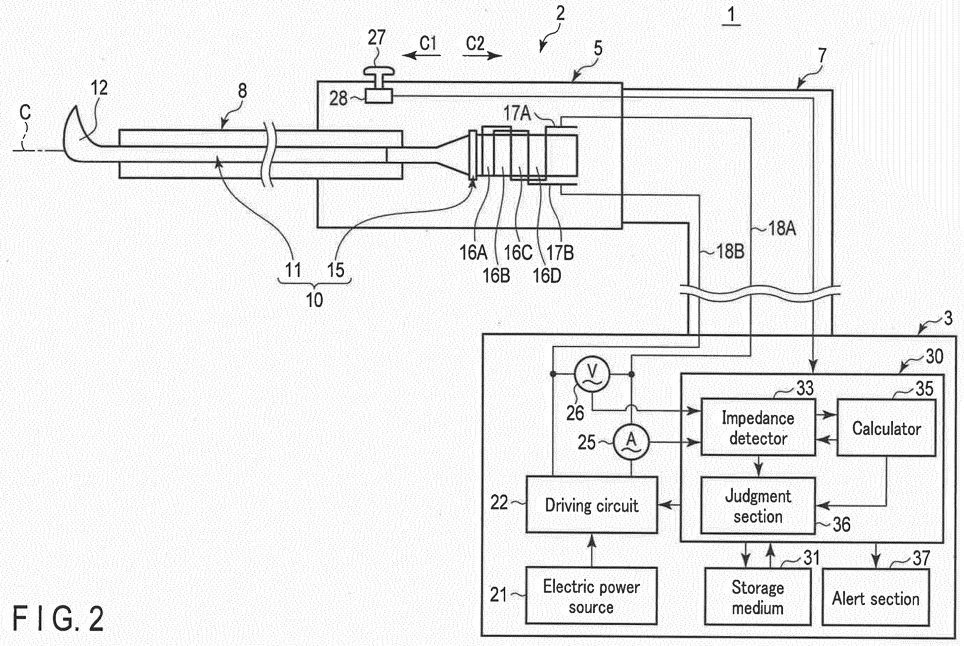

FIG. 2 is a view illustrating the configuration of the treatment instrument 2 and energy control device 3. As illustrated in FIG. 2, the treatment instrument 2 has a longitudinal axis C. Here, one side of a direction along the longitudinal axis C is defined as a distal side (arrow C1 side in FIG. 1), and a side opposite to the distal side is defined as a proximal side (direction of arrow C2 in FIG. 1). In the treatment instrument 2, the housing 5 extends along the longitudinal axis C.

A sheath 8, which extends along the longitudinal axis C, is coupled to the housing 5. The sheath 8 is coupled to the housing 5 in the state in which the sheath 8 is inserted in the inside of the housing 5 from the distal side. From the inside of the housing 5, a vibration transmitting member 11 extends toward the distal side along the longitudinal axis (with the longitudinal axis C as the center) through the inside of the sheath 8. An end effector (treatment portion) 12 is formed at a distal portion of the vibration transmitting member 11. The vibration transmitting member 11 is inserted through the sheath 8 in the state in which the end effector 12 projects to the distal side from the distal end of the sheath 8. Incidentally, in the present embodiment, although the end effector 12 is formed in a hook shape, the end effector 12 may be formed in a shape other than the hook shape, such as a spatula shape or a blade shape.

A vibration generator (ultrasonic transducer) 15 is provided in the inside of the housing 5. In the inside of the housing 5, the vibration transmitting member 11 is connected to the distal side of the vibration generator 15. The vibration generator 15 includes piezoelectric elements 16A to 16D (four piezoelectric elements in this embodiment), and ultrasonic electrodes 17A and 17B. The energy control device 3 includes an electric power source 21 and a driving circuit (output section) 22. The ultrasonic electrode 17A is electrically connected to the driving circuit 22 via an electrical path 18A which extends through the inside of the cable 7, and the ultrasonic electrode 17B is electrically connected to the driving circuit 22 via an electrical path 18B which extends through the inside of the cable 7.

The electric power source 21 is a battery or a plug socket. In the driving circuit 22, electric power from the electric power source 21 is converted to ultrasonic electric energy (AC electric power). By the converted ultrasonic electric energy being output from the driving circuit 22, the ultrasonic electric energy is supplied to the vibration generator 15 via the electrical paths 18A and 18B. Thereby, a driving voltage (AC voltage) V is applied to the piezoelectric elements 16A to 16D between the ultrasonic electrodes 17A and 17B, and a driving current (AC current) I flows through the piezoelectric elements 16A to 16D. Specifically, the driving current (ultrasonic current) I, which was output from the driving circuit (output section) 22, is supplied to the piezoelectric elements 16A to 16D of the vibration generator 15. In the present embodiment, by the driving current I being supplied from the driving circuit 22 to the vibration generator 15, the treatment instrument 2 is driven.

By the treatment instrument 2 being driven by the driving current (ultrasonic current) I, the driving current I is converted to ultrasonic vibration by the piezoelectric elements 16A to 16D, and ultrasonic vibration is generated. The generated ultrasonic vibration is transmitted from the vibration generator 15 to the vibration transmitting member 11, and is transmitted from the proximal side to the distal side in the vibration transmitting member 11. Thereby, the ultrasonic vibration is transmitted to the end effector 12, and the end effector 12 performs a treatment by using the ultrasonic vibration. In the present embodiment, for example, as illustrated in FIG. 1, the end effector 12 is put in contact with a bone or a cartilage in a joint cavity 70, and the end effector 12 abrades the bone or cartilage as a treated target by using the transmitted ultrasonic vibration. Here, in the state in which the ultrasonic vibration generated by the vibration generator 15 is being transmitted to the end effector 12, a vibrating body 10 is formed by the vibration generator 15 and vibration transmitting member 11. By the vibrating body 10 transmitting the ultrasonic vibration toward the end effector 12, the vibrating body 10 longitudinally vibrates in a vibration direction parallel to the longitudinal axis C in a predetermined frequency range (e.g. in a range of 46 kHz to 48 kHz).

In the energy treatment system 1, there is provided a current detector 25, such as an amperemeter, which detects, with the passing of time, the driving current I which is output from the driving circuit (output section) 22 (i.e. which is supplied to the vibration generator 15). The current detector 25 is configured to detect the driving current I flowing in the electrical path (18A or 18B). In addition, in the energy treatment system 1, there is provided a voltage detector 26, such as a voltmeter, which detects, with the passing of time, the driving voltage (ultrasonic voltage) V which is applied between the ultrasonic electrodes 17A and 17B (between the electrical paths 18A and 18B) by the output of the driving current I from the driving circuit 22. The voltage detector 26 is configured to detect the driving voltage V (electric potential difference) between the electrical paths 18A and 18B.

An energy operation button 27, which is an energy operation input portion, is attached to the housing 5. In addition, a switch 28 is provided in the inside of the housing 5. The open or closed state of the switch 28 changes based on the presence or absence of an input of an energy operation in the energy operation button 27. In the energy control device 3, a controller 30 and a storage medium 31 such as a memory are provided. The controller 30 includes a processor or an integrated circuit, which includes, for example, a CPU (Central Processing Unit) or an ASIC (application specific integrated circuit), and the controller 30 can store information or the like in the storage medium 31, and can read the information or the like stored in the storage medium 31. In addition, the controller 30 may be composed of a single processor, or the controller 30 may be composed of a plurality of processors. By detecting the open or closed state of the switch 28, the controller 30 detects whether the energy operation is input in the energy operation button 27. Furthermore, based on the input of the energy operation in the energy operation button 27, the controller 30 controls the output of the driving current I (ultrasonic electric energy) from the driving circuit 22.

The controller 30 includes an impedance detector 33, a calculator 35 and a judgment section 36. The impedance detector 33, calculator 35 and judgment section 36 execute, for example, some of processes which are executed by processors that constitute the controller 30. The calculator 35 executes an arithmetic process, based on detection results of the current detector 25 and voltage detector 26. For example, a phase difference $\varphi$ between the driving current I and driving voltage V is calculated with the passing of time by the calculator 35.

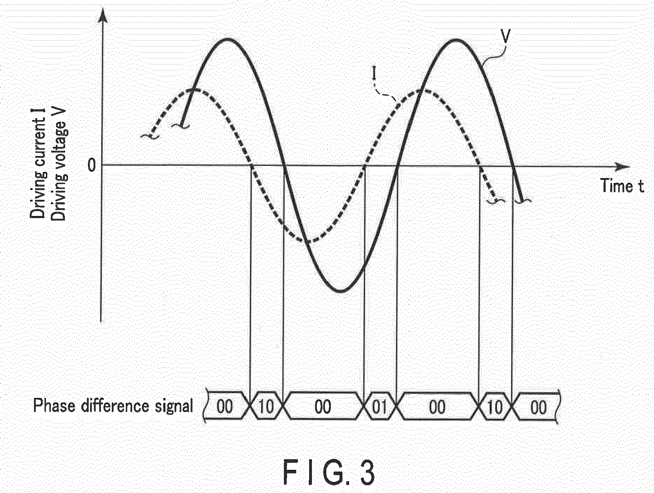

FIG. 3 is a view for describing a process of calculating the phase difference $\varphi$ between the driving current I and driving voltage V, the process being executed by the calculator 35. The graph of FIG. 3 shows an example of variations with time of the driving current I and driving voltage V. The abscissa indicates time t, with the start of output of the driving current I being set as a reference. The ordinate indicates the driving current I and driving voltage V. In addition, in the graph of FIG. 3, a variation with time of the driving current I is indicated by a broken line, and a variation with time of the driving voltage V is indicated by a solid line. Based on the variations with time of the driving current I and driving voltage V, the calculator 35 continuously generates, with the passing of time, a phase difference signal indicative of the phase difference $\varphi$ between the driving current I and driving voltage V. As illustrated in FIG. 3, when both the driving current I and driving voltage V are positive, and when both the driving current I and driving voltage V are negative, the signal value of the phase difference signal becomes "00". In addition, when the driving current I is negative and the driving voltage V is positive, the signal value of the phase difference signal becomes "10". When the driving current I is positive and the driving voltage V is negative, the signal value of the phase difference signal becomes "01". The calculator 35 calculates the phase difference $\varphi$ between the driving current I and driving voltage V, for example, based on the ratio of a time during which the signal value of the phase difference signal becomes "10" in a half cycle of the driving current I, or the ratio of a time during which the signal value of the phase difference signal becomes "01" in the half cycle of the driving current I. In this case, the phase difference $\varphi$ is calculated with the passing of time in every half cycle of the driving current I.

The impedance detector 33 detects, with the passing of time, an acoustic impedance (ultrasonic impedance) Z of ultrasonic electric energy (i.e. the vibration generator (ultrasonic transducer) 15), based on the detection results in the current detector 25 and voltage detector 26, and the calculation result of the phase difference $\varphi$. The acoustic impedance Z is calculated as indicated by equation (1), based on the driving current (ultrasonic current) I, driving voltage (ultrasonic voltage) V and phase difference $\varphi$.

$$Z = V/I \times \cos\varphi \qquad\qquad (1)$$

Here, for example, the calculation of the acoustic impedance Z using equation (1) is executed, for example, by setting a peak-to-peak value Ipp of the driving current (AC current) I as a current value and setting a peak-to-peak value Vpp of the driving voltage (AC voltage) V as a voltage value. However, the acoustic impedance Z may be calculated by setting a crest value (maximum value) Im of the driving current I as a current value and setting a crest value (maximum value) Vm of the driving voltage V as a voltage value, and the acoustic impedance Z may be calculated by setting an effective value Ie of the driving current I as a current value and setting an effective value Ve of the driving voltage V as a voltage value.

The judgment section 36 judges, based on the detection results of the driving current I, driving voltage V and acoustic impedance Z and the calculation result in the calculator 35, whether the vicinity of a treated target (bone or cartilage), with which the end effector 12 is in contact, is filled with a liquid in the joint cavity (70), in the state in which the end effector 12 is in contact with the treated target and the driving current I is being output from the driving circuit 22 (in the state in which the vibrating body 10 is transmitting ultrasonic vibration). Thereby, it is judged whether the contact part of the end effector 12, which is in contact with the treated target, is located in liquid or not. If the judgment section 36 judged that the vicinity of the treated target is not filled with liquid, the judgment section 36 generates a signal indicating that the vicinity of the treated target is not filled with liquid. Based on a judgment result in the judgment section 36 (as to whether the vicinity of the treated target is filled with liquid or not), the controller 30 controls the output of the driving current I (ultrasonic electric energy) from the driving circuit (output section) 22.

In addition, in the energy control device 3, an alert section 37, the actuation of which is controlled by the controller 30, is provided. The alert section 37 is a lamp, a buzzer, a monitor or the like, and is actuated to issue an alert.

Next, the function and the advantageous effects of the energy treatment system 1 and energy control device 3 will be described. When a bone or cartilage is abraded as a treated target by using the energy treatment system 1 (treatment instrument 2), the end effector 12 is inserted in a body cavity such as the joint cavity 70. At this time, as illustrated in FIG. 1, the distal portion of the endoscope (rigid endoscope) 50 and the distal portion of the liquid supply assisting instrument 60 are also inserted in the joint cavity 70. Then, by imaging the subject by the imaging element (not shown) of the endoscope 50, the inside of the joint cavity 70 including the treated target is observed. In addition, in the joint cavity 70, a liquid is jetted from the jet port 62 of the liquid supply assisting instrument 60, and a liquid L such as physiological saline is supplied into the joint cavity 70. Incidentally, instead of providing the liquid supply assisting instrument 60, a liquid supply channel may be formed in the inside of the sheath 8 of the treatment instrument 2, and liquid (L) may be supplied into the joint cavity 70 through the liquid supply channel of the treatment instrument 2. In addition, a suction channel may be provided in the endoscope 50, and liquid (L) may be discharged from the inside of the joint cavity 70 to the outside of the body through the suction channel.

When the end effector 12 is inserted in the body cavity, a surgeon inputs an energy operation by the energy operation button 27. Thereby, by the controller 30, the driving current I (ultrasonic electric energy) is output from the driving circuit 22, and ultrasonic vibration is generated from the supplied driving current I in the vibration generator 15. The generated ultrasonic vibration is transmitted to the end effector 12. In the state in which the end effector 12 longitudinally vibrates by the ultrasonic vibration, the end effector 12 is put in contact with the treated target, and thereby the treated target (bone or cartilage) is abraded.

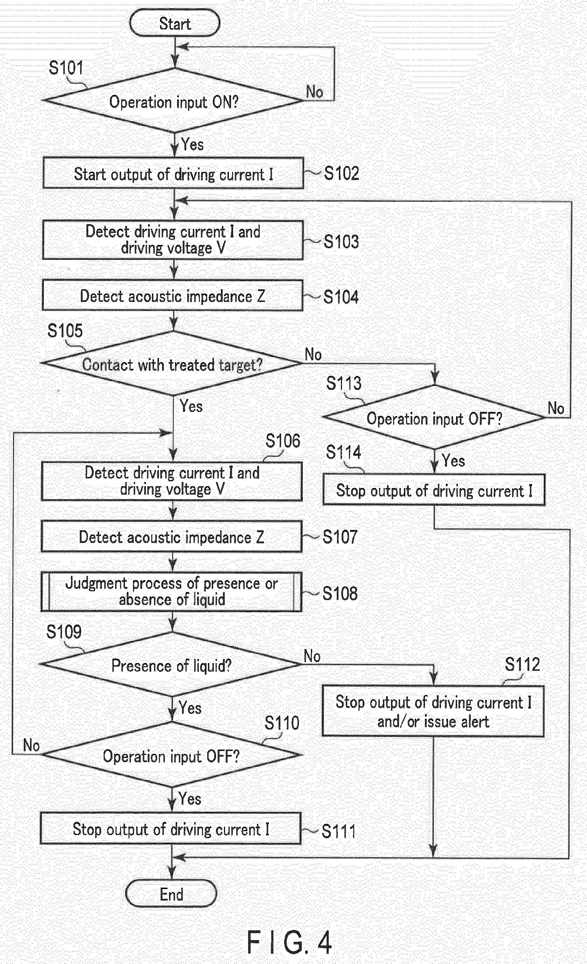

FIG. 4 is a flowchart illustrating a process in the energy control device 3 at a time of performing a treatment by using the energy treatment system 1. As illustrated in FIG. 4, when the treatment of abrading the bone or cartilage is performed, the controller 30 detects whether the energy operation is input by the energy operation button 27 (step S101). Unless the input of the energy operation is detected (step S101-No), the process returns to step S101. If the input of the energy operation is detected (step S101-Yes), the controller 30 starts the output of the driving current I (ultrasonic electric energy) from the driving circuit (energy output section) 22 (step S102). Thereby, as described above, ultrasonic vibration is transmitted to the end effector 12.

In the present embodiment, in the state in which the driving current I is being output, the controller 30 controls the output of the driving current I in such a state that the current value of the driving current I is kept at a constant reference current value (current value) Iref with the passing of time. Here, the amplitude of the vibrating body 10 including the end effector 12 is proportional to the current value of the driving current I. Thus, by the current value of the driving current I being kept constant with the passing of time, the vibrating body 20 (end effector 12) vibrates at a constant amplitude with the passing of time. In addition, if the output of the driving current I is started, the current detector 25 detects the driving current I with the passing of time, and the voltage detector 26 detects the driving voltage V with the passing of time (step S103). In addition, based on the detected driving current I and driving voltage V, the impedance detector 33 detects the acoustic impedance Z with the passing of time (step S104). The acoustic impedance Z is calculated by using the above equation (1), by calculating, as described above, the phase difference $\varphi$ between the driving current I and driving voltage V. The acoustic impedance Z is calculated periodically, for example, in every cycle or every half cycle of the driving current I.

In the constant current control in which the driving current I is kept constant at the reference current value Iref with the passing of time, if the acoustic impedance Z increases, the voltage value of the driving voltage V is increased and the current value of the driving current I is kept constant. Conversely, if the acoustic impedance Z decreases, the voltage value of the driving voltage V is decreased and the current value of the driving current I is kept constant. In the present embodiment, the constant current control, in which the current value is kept constant at the reference current value Iref with the passing of time, is executed by setting the peak-to-peak value Ipp of the driving current (AC current) I as the current value. However, although the value of the reference current value Iref is different from the case in which the peak-to-peak value Ipp is set as the current value, the above-described constant current control may be executed by setting the crest value (maximum value) Im of the driving current I as the current value, and the above-described constant current control may be executed by setting the effective value Ie of the driving current I as the current value.

When the acoustic impedance Z is detected, the judgment section 36 judges whether the end effector 12 is in contact with the treated target (step S105). The judgment as to whether the end effector 12 is in contact with the treated target is executed, for example, based on the variations with time of the acoustic impedance Z and driving voltage V. In a certain example, it is judged that the end effector 12 is in contact with the treated target, based on the fact that an acoustic impedance Z(t) at time t, which is detected by the impedance detector 33, has increased to an impedance threshold (first acoustic impedance threshold) Zth1 or more. Here, the time t is a variable, with the start of output of the driving current I being set as a reference. In addition, in a certain example, it may be judged that the end effector 12 is in contact with the treated target, based on the fact that the acoustic impedance Z has continuously increased during a reference time at an increase rate of a reference increase rate or more. In another certain example, it may be judged that the end effector 12 is in contact with the treated target, based on the fact that the voltage value of the driving voltage V(t) at time t has increased to a voltage threshold (first driving voltage threshold) Vth1 or more.

If the judgment section 36 judged that the end effector 12 is not in contact with the treated target (step S105-No), the controller 30 detects whether the input of the energy operation by the energy operation button 27 is continued or not (step S113). If the input of the energy operation is stopped (step S113-Yes), the controller 30 stops the output of the driving current I from the driving circuit 22 (step S114). If the input of the energy operation is continued (step S113-No), the process returns to step S103, and the above-described process of step S103 onwards is successively executed.

In step S105, if the judgment unit 36 judged that the end effector 12 is in contact with the treated target (step S105-Yes), the current detector 25 continuously detects the driving current I with the passing of time, and the voltage detector 26 continuously detects the driving voltage V with the passing of time (step S106). In addition, based on the detected driving current I and driving voltage V, the impedance detector 33 continuously detects the acoustic impedance Z with the passing of time (step S107). At this time, too, by the controller 30, the current value of the driving current I is kept at the constant reference current value (current value) Iref with the passing of time, and the vibrating body 20 vibrates at the constant amplitude with the passing of time.

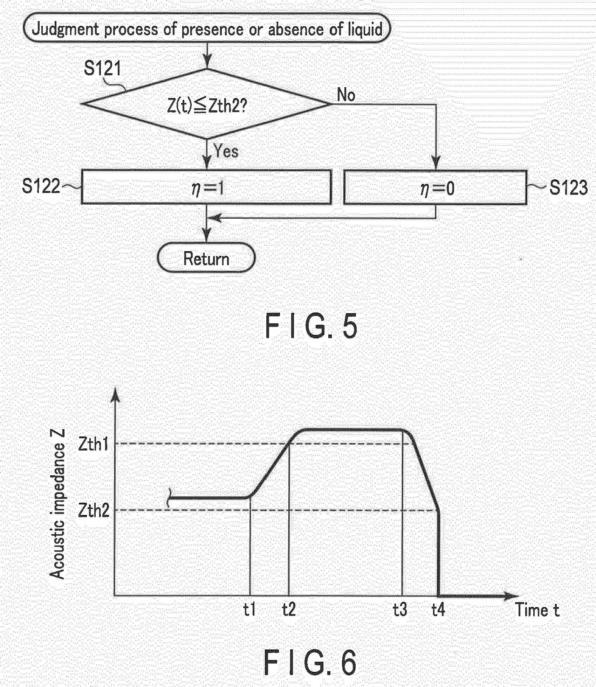

When the acoustic impedance Z is detected, the judgment unit 36 executes a judgment process of the presence or absence of liquid in the vicinity of the treated target, based on the detection result of the acoustic impedance Z (step S108). FIG. 5 is a flowchart illustrating the judgment process of the presence or absence of liquid in the vicinity of the treated target, the judgment process being executed by the energy control device 3. As illustrated in FIG. 5, in the judgment process of the presence or absence of liquid (the judgment process of judging whether liquid is filled or not), the judgment section 36 judges whether or not the acoustic impedance Z(t) at the time t, which was detected by the impedance detector 33, was an impedance threshold (second acoustic impedance threshold) Zth2 or less (step S121). The impedance threshold Zth2 is less than the impedance threshold Zth1, and is, for example, in a range of 500 Ω to 1200 Ω. In addition, the impedance threshold Zth2 may be set by the surgeon or the like, and may be stored in the storage medium 31. If the acoustic impedance Z(t) was the impedance threshold (second acoustic impedance threshold) Zth2 or less (step S121-Yes), the judgment section 36 sets a judgment parameter η to 1 (step S122). On the other hand, if the acoustic impedance Z(t) was greater than the impedance threshold Zth2 (step S121-No), the judgment section 36 sets the judgment parameter η to 0 (step S123).

As illustrated in FIG. 4 and FIG. 5, based on the judgment parameter η that was set in the judgment process of the presence or absence of liquid (step S108), the judgment section 36 judges whether the vicinity of the treated target, with which the end effector 12 is in contact, is filled with liquid or not (step S109). Thereby, it is judged, with the passing of time, whether the contact part of the end effector 12, which is in contact with the treated target, is located in liquid (L) or not. Specifically, it is judged, with the passing of time, whether the end effector 12 is immersed in liquid or not. When the judgment parameter η was set to 0, the judgment section 36 judges that the vicinity of the treated target, with which the end effector 12 is in contact, is filled with liquid (step S109-Yes). On the other hand, when the judgment parameter η was set to 1, the judgment section 36 judges that the vicinity of the treated target is not filled with liquid (i.e. the contact part of the end effector 12, which is in contact with the treated target, is located in the air) (step S109-No). Accordingly, in the present embodiment, based on whether or not the acoustic impedance Z has decreased to the impedance threshold (second acoustic impedance threshold) Zth2 or less, it is judged whether the vicinity of the treated target, with which the end effector 12 is in contact, is filled with liquid or not (whether liquid is properly present in the vicinity of the treated target).

If the judgment section 36 judged that liquid is filled (step S109-Yes), the controller 30 detects whether the input of the energy operation by the energy operation button 27 is continued or not (step S110). If the input of the energy operation is stopped (step S110-Yes), the controller 30 stops the output of the driving current I from the driving circuit 22 (step S111). If the input of the energy operation is continued (step S110-No), the process returns to step S106, and the above-described process of step S106 onwards is successively executed.

In step S109, when it was judged that the vicinity of the treated target is not filled with liquid (step S109-No), the judgment section 36 generates a signal indicating that liquid is not filled (i.e. the contact part of the end effector 12, which is in contact with the treated target, is located in the air). If the signal indicating that the vicinity of the treated target is not filled with liquid is generated, the controller 30 stops the output of the driving current I from the driving circuit 22 (step S112). Incidentally, the case of stopping the output includes not only a case of completely stopping the output of the driving current I, but also a case of passing a weak driving current I of such a magnitude as not to affect the treatment. In addition, instead of stopping the output of the driving current I, it is possible to cause the alert section 37 to issue an alert to the effect that liquid is not filled. Needless to say, in addition to stopping the output of the driving current I, it is possible to cause the alert section 37 to issue an alert to the effect that liquid is not filled (step S112). The alert section 37 issues an alert, for example, by turning on a light, producing sound, or monitor-displaying an alert.

FIG. 6 is a view illustrating an example of a variation with time of the acoustic impedance Z. In FIG. 6, the abscissa indicates time t, with the start of output of the driving current I being set as a reference, and the ordinate indicates the acoustic impedance Z. As illustrated in FIG. 6, when a bone or cartilage is abraded as a treated target, the end effector 12 begins to come in contact with the treated target, and thereby the acoustic impedance Z increases with time. Thus, in the state in which the end effector 12 is not in contact with the treated target, the acoustic impedance Z is small. In an example illustrated in FIG. 6, at time t1 or thereabout, the end effector 12 begins to come in contact with the treated target, and the treated target begins to be abraded. Then, from the time t1 and thereabout, the acoustic impedance Z begins to increase with time. In the meantime, in the example illustrated in FIG. 6, before the end effector 12 begins to come in contact with the treated target at time t1 or thereabout, the end effector 12 is vibrating in the liquid in a state of non-contact with the treated target.

In the example illustrated in FIG. 6, before time t2, the process of steps S103 to S105 in FIG. 4 is repeated with the passing of time. Then, at time t2, if the acoustic impedance Z(t) increases to the impedance threshold (first acoustic impedance threshold) Zth1 or more, it is judged, by the process of step S105, that the end effector 12 is in contact with the treated target. If it is judged at time t2 that the end effector 12 is in contact with the treated target, the process of steps S106 to S109 in FIG. 4 is executed. Accordingly, before the time t2 in the example shown in FIG. 6 (in the state in which the end effector 12 is vibrating in the liquid in a state of non-contact with the treated target), the process of steps S106 to S109 is not executed.

While the treated target is being abraded in the state in which the contact part of the end effector 12, which is in contact with the treated target, is located in the liquid, there may be a case in which the liquid is not properly supplied to the vicinity of the treated target. In this case, the vicinity of the treated target is not filled with liquid, and the end effector 12 cuts the treated target (bone or cartilage) in the state in which the contact part of the end effector 12, which is in contact with the treated target, is located in the air (in the state in which the contact part of the end effector 12, which is in contact with the treated target, is not located in the liquid).

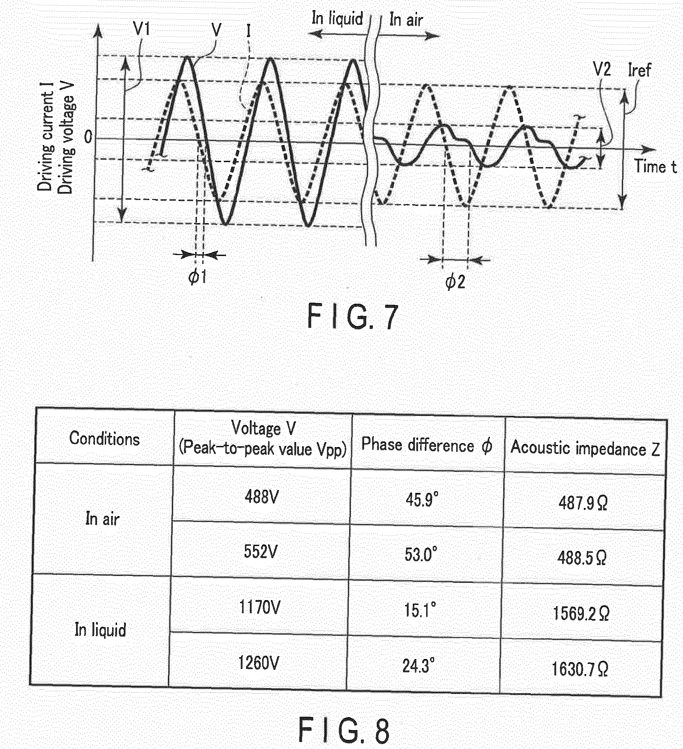

FIG. 7 illustrates an example of variations with time of the driving voltage V and driving current I in the state in which the end effector 12 located in the liquid is abrading the treated target, and in the state in which the end effector 12 located in the air is abrading the treated target. In FIG. 7, the abscissa indicates time t, with the start of output of the driving current I being set as a reference, and the ordinate indicates the driving current I and driving voltage V. In addition, in FIG. 7, a variation with time of the driving current I is indicated by a broken line, and a variation with time of the driving voltage V is indicated by a solid line. In the present embodiment, the above-described constant current control, in which the current value of the driving current I (peak-to-peak value Ipp in this embodiment) is kept constant at the reference current value Iref with the passing of time, is executed. Thus, both in the state in which the end effector 12 is located in the liquid and in the state in which the end effector 12 is located in the air, the current value of the driving current I is kept constant at the reference current value Iref with the passing of time.

On the other hand, compared to the state in which the end effector 12 is located in the liquid (the state in which the vicinity of the treated target is filled with liquid), in the state in which the end effector 12 is located in the air (the state in which the vicinity of the treated target is not filled with liquid), the voltage value of the driving voltage V (the peak-to-peak value Vpp in this embodiment) becomes smaller. In the example illustrated in FIG. 7, in the state in which the end effector 12 located in the liquid is abrading the treated target, the driving voltage V has a voltage value (first voltage value) V1. On the other hand, in the state in which the end effector 12 located in the air is abrading the treated target, the driving voltage V has a voltage value (second voltage value) V2 which is less than the voltage value (first voltage value) V1.

In addition, compared to the state in which the end effector 12 is located in the liquid (the state in which the vicinity of the treated target is filled with liquid), in the state in which the end effector 12 is located in the air (the state in which the vicinity of the treated target is not filled with liquid), the phase difference $\varphi$ between the driving current I and driving voltage V becomes greater. In the example illustrated in FIG. 7, in the state in which the end effector 12 located in the liquid is abrading the treated target, the driving current I has a phase difference (first phase difference) $\varphi$1 relative to the driving voltage V. On the other hand, in the state in which the end effector 12 located in the air is abrading the treated target, the driving current I has a phase difference (second phase difference) $\varphi$2, which is greater than the phase difference $\varphi$1, relative to the driving voltage V.

As described above, compared to the state in which the end effector 12 is located in the liquid, in the state in which the end effector 12 is located in the air, the voltage value of the driving voltage V becomes smaller, and the phase difference $\varphi$ between the driving current I and driving voltage V becomes greater. Accordingly, from the above-described equation (1), the acoustic impedance Z becomes smaller in the state in which the end effector 12 is located in the air (the state in which the vicinity of the treated target is not filled with liquid), than in the state in which the end effector 12 is located in the liquid (the state in which the vicinity of the treated target is filled with liquid).

FIG. 8 illustrates measurement data of the voltage value (peak-to-peak value Vpp) of the driving voltage V, phase difference $\varphi$ and acoustic impedance Z in the state in which the end effector 12 located in the liquid is abrading the treated target, and in the state in which the end effector 12 located in the air is abrading the treated target. In FIG. 8, measurement was conducted twice, in each of the state in which the end effector 12 located in the liquid is abrading the treated target, and the state in which the end effector 12 located in the air is abrading the treated target. In the measurement, both in the state in which the end effector 12 is located in the liquid and in the state in which the end effector 12 is located in the air, the same end effector 12 was used, and the treated target that is to be abraded was the same. Furthermore, both in the state in which the end effector 12 is located in the liquid and in the state in which the end effector 12 is located in the air, the constant current control, in which the current value (peak-to-peak value Ipp) of the driving current I is kept constant at 0.72 A with the passing of time, was executed.

Also in the measurement illustrated in FIG. 8, the voltage values (488 V and 552 V) of the driving voltage V in the state in which the end effector 12 is located in the air became lower than the voltage values (1170 V and 1260 V) of the driving voltage V in the state in which the end effector 12 is located in the liquid. In addition, also in the measurement illustrated in FIG. 8, the phase difference $\varphi$ (45.9° and 53.0°) between the driving current I and driving voltage V in the state in which the end effector 12 is located in the air became greater than the phase difference $\varphi$ (15.1° and 24.3°) between the driving current I and driving voltage V in the state in which the end effector 12 is located in the liquid. Thus, in the measurement of FIG. 8, too, the acoustic impedances Z (487.9 $\Omega$ and 488.5 $\Omega$) in the state in which the end effector 12 is located in the air became smaller than the acoustic impedances Z (1568.2 Ω and 1630.7 Ω) in the state in which the end effector 12 is located in the liquid.

In addition, in the example illustrated in FIG. 6, at time t3 or thereabout after time t2, there occurs a state in which the vicinity of the treated target with which the end effector 12 is in contact (i.e. which is being cut by the end effector 12) is not filled with liquid. Thus, from the time t3 or thereabout, the acoustic impedance Z begins to decrease with time. In the example illustrated in FIG. 6, in the state in which the acoustic impedance Z after the time t3 is decreasing with time, the process of steps S106 to S109 in FIG. 4 is repeatedly executed with the passing of time. Then, at time 4, it is judged that the acoustic impedance Z(t) has decreased to the impedance threshold (second acoustic impedance threshold) Zth2 or less, and, at the time 4 or immediately thereafter, it is judged by the process of step S109 that the vicinity of the treated target is not filled with liquid (the contact part of the end effector 12, which is in contact with the treated target, is not located in the liquid). Then, the signal indicating that liquid is not filled is generated, and at the time 4 or immediately thereafter, the output of the driving current I is stopped and/or an alert is issued by the alert section 37 by the process of step S112.

In the present embodiment, based on whether or not the acoustic impedance Z has decreased to the impedance threshold (second acoustic impedance threshold) Zth2 or less, it is judged whether the vicinity of the treated target, with which the end effector 12 is in contact, is filled with liquid or not. As described above, in the state in which the vicinity of the treated target is not filled with liquid (i.e. the contact part of the end effector 12, which is in contact with the treated target, is located in the air), the acoustic impedance Z decreases. Thus, based on whether or not the acoustic impedance Z(t) at time t was the impedance threshold Zth2 or less, it is properly judged whether the vicinity of the treated target, with which the end effector 12 is in contact, is filled with liquid or not.

In addition, in this embodiment, if it is judged that the vicinity of the treated target is not filled with liquid (the contact part of the end effector 12, which is in contact with the treated target, is not located in the liquid), the output of the driving current I is stopped and/or the alert is issued. By the current I being automatically stopped, or by the surgeon stopping the input of the energy operation by the alert, the ultrasonic vibration is prevented from being transmitted to the end effector 12. Thereby, in the state in which the vicinity of the treated target is not filled with liquid (the contact part of the end effector 12, which is in contact with the treated target, is not located in the liquid), the treated target is effectively prevented from being abraded by the ultrasonic vibration. Accordingly the amount of heat produced in the vicinity of the treated target is suppressed to be small, and the treatment performance in the treatment using ultrasonic vibration can be secured.

Additionally, in the present embodiment, before the acoustic impedance Z increases to the impedance threshold (first acoustic impedance threshold) Zth1 or more, it is not executed to judge whether the vicinity of the treated target is filled with liquid or not. Specifically, after a change from the state in which the end effector 12 is not in contact with the treated target in the liquid to the state in which the end effector 12 has come in contact with the treated target, it is judged whether the vicinity of the treated target is filled with liquid or not. Thus, when the end effector 12 is vibrating in the liquid in the state in which the end effector 12 is not in contact with the treated target, the judgment section 36 is effectively prevented from erroneously judging that the vicinity of the treated target is not filled with liquid.

In the meantime, compared to the state in which the end effector 12, which is not in contact with the treated target, is vibrating in the liquid, in the state in which the end effector 12, which is in contact with the treated target, is vibrating in the air, the acoustic impedance Z becomes smaller. Thus, if the impedance threshold (second acoustic impedance threshold) Zth2, which is used in the judgment process of the presence or absence of liquid in the vicinity of the treated target, is set to be lower than the acoustic impedance Z in the state in which the end effector 12, which is not in contact with the treated target, is vibrating in the liquid, there is no need to execute the process of steps S103 to S105 in FIG. 4. In this case, the impedance threshold Zth2 is set, for example, in a range of 500 Ω to 550 Ω.

(Modifications of First Embodiment)

In the meantime, in the first embodiment, based on the fact that the acoustic impedance Z has decreased to the impedance threshold (second acoustic impedance threshold) Zth2 or less, it is judged that the vicinity of the treated target (bone or cartilage), with which the end effector 12 is in contact, is not filled with liquid. However, the restriction to this is unnecessary. For example, in a certain modification, the judgment process (step S108 in FIG. 4) of the presence or absence of liquid in the vicinity of the treated target may be executed by using the voltage threshold (second driving voltage threshold) Vth2 in place of the impedance threshold Zth2. In this case, based on whether or not the voltage value of the driving voltage V(t) at time t was the voltage threshold Vth2 or less, it is judged whether the vicinity of the treated target, with which the end effector 12 is in contact, is filled with liquid or not. Specifically, if the voltage value of the driving voltage V(t) was the voltage threshold Vth2 or less, the judgment unit 36 sets a judgment parameter η to 1, and it is judged in step S109 that the vicinity of the treated target is not filled with liquid (the contact part of the end effector 12, which is in contact with the treated target, is located in the air) (step S109-No). On the other hand, if the voltage value of the driving voltage V(t) was greater than the voltage threshold Vth2, the judgment unit 36 sets the judgment parameter η to 0, and it is judged in step S109 that the vicinity of the treated target is filled with liquid (the contact part of the end effector 12, which is in contact with the treated target, is located in the liquid) (step S109-Yes). Incidentally, in the present modification, too, the judgment process (step S108) of the presence or absence of liquid in the vicinity of the treated target is not executed before it is judged in step S105 in FIG. 4 that the end effector 12 is in contact with the treated target.

In the present modification, too, in the state in which the driving current I is being output from the driving circuit (output unit) 22, the above-described constant current control, in which the current value of the driving current I is kept constant (at the reference current value Iref) with the passing of time, is executed. As described in the first embodiment, when the constant current control of the driving current I is executed, the voltage value of the driving voltage V becomes smaller in the state in which the end effector 12 is located in the air (the state in which the vicinity of the treated target is not filled with liquid), than in the state in which the end effector 12 is located in the liquid (the state in which the vicinity of the treated target is filled with liquid). Thus, based on whether or not the voltage value of the driving voltage V(t) at time t was the voltage threshold (second driving voltage threshold) Vth2 or less, it is properly judged whether the vicinity of the treated target, with which the end effector 12 is in contact, is filled with liquid or not. Incidentally, as the voltage value of the driving voltage (AC voltage) V, the peak-to-peak value Vpp, crest value (maximum value) Vm, or effective value Ve is used. However, depending on which of the peak-to-peak value Vpp, crest value Vm and effective value Ve is used as the voltage value, the value of the voltage threshold Vth2 varies. Here, when the peak-to-peak value Vpp of the driving voltage V is used as the voltage value, the voltage threshold Vth2 is set, for example, in a range of 700 V to 1000 V.

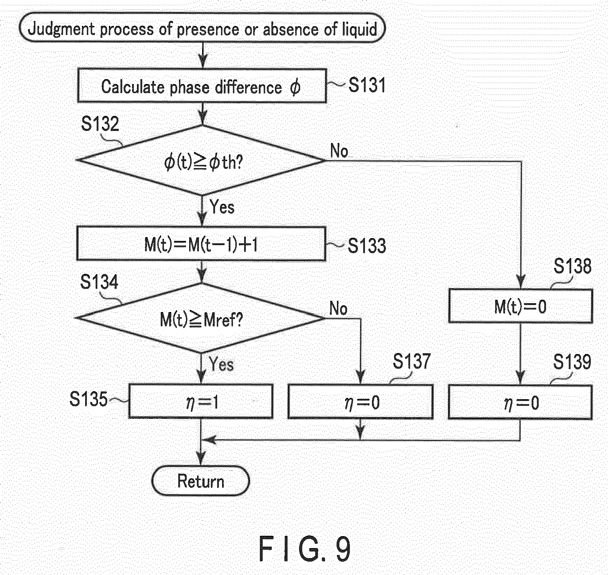

Additionally, in a first modification of the first embodiment, which is illustrated in FIG. 9, it is judged whether the vicinity of the treated target is filled with liquid or not, based on the phase difference φ between the driving current I and driving voltage V. Incidentally, in the present modification, the process of step S107 in FIG. 4 (the detection of acoustic impedance Z with the passing of time) may not be executed. In addition, in this modification, too, the judgment process (step S108) of the presence or absence of liquid in the vicinity of the treated target is not executed before it is judged in step S105 in FIG. 4 that the end effector 12 is in contact with the treated target.

FIG. 9 is a flowchart illustrating the judgment process (the process of step S108 in FIG. 4) of the presence or absence of liquid in the vicinity of a treated target, the judgment process being executed by the energy control device 3. As illustrated in FIG. 9, in the judgment process of the presence or absence of liquid, the calculator 35 calculates a phase difference φ(t) at time t between the driving current I and driving voltage V, based on the detected driving current I and driving voltage V (step S131). The phase difference φ(t) is calculated, for example, as described above in the first embodiment, and is calculated, for example, in every half cycle or every cycle of the driving current I. If the phase difference φ(t) at time t is calculated, the judgment section 36 judges whether or not the calculated phase difference φ(t) was a phase threshold φth or more (step S132). Incidentally, the phase threshold φth is set, for example, in a range of 30° to 40°, and may be set by the surgeon or the like, and may be stored in the storage medium 31.

Here, a count number M(t) at time t is specified. If the phase difference φ(t) was the phase threshold φth or more (step S132-Yes), the calculator 35 calculates the count number M(t) at time t, by adding 1 to a count number M(t-1) at a time point (t-1) which is a detected object in immediately previous detection prior to the time t (step S133). At this time, if the count number M(t-1) at time (t-1) is 1, the count number M(t) at time t becomes 2. The calculated count number M(t) is stored in the storage medium 31. Incidentally, at the time point when it was judged in step S105 in FIG. 4 that the end effector 12 is in contact with the treated target (and at the time of start of output of driving current I), the count number M(t) is set at 0.

If the count number M(t) is calculated in step S133, the judgment section 36 judges whether the calculated count number M(t) was a reference count number Mref or more (step S134). Here, the reference count number Mref may be set by the surgeon or the like, and may be stored in the storage medium 31. In addition, if the count number M(t) was the reference count number Mref or more (step S134-Yes), the judgment section 36 sets the judgment parameter η to 1 (step S135). Then, in step S109 in FIG. 4, the judgment section 36 judges that the vicinity of the treated target, with which the end effector 12 is in contact, is not filled with liquid (step S109-No).

On the other hand, if the count number M(t) was less than the reference count number Mref (step S134-No), the judgment section 36 sets the judgment parameter η to 0 (step S137). Then, in step S109 in FIG. 4, the judgment unit 36 judges that the vicinity of the treated target is filled with liquid (step S109-Yes). In this case, however, a value, which was obtained by adding 1 to the count number M(t-1) at the time (t-1), is stored as the count number M(t) at time t. In addition, in step S132, if the phase difference φ(t) at time t was less than the phase threshold φth (step S132-No), the calculator 35 resets the count number M(t) at time t to 0 (step S138). Then, the judgment section 36 sets the judgment parameter η to 0 (step S139), and, in step S109 in FIG. 4, the judgment section 36 judges that the vicinity of the treated target is filled with liquid (step S109-Yes). At this time, the reset value (i.e. 0) is stored as the count number M(t) at time t.

The judgment process (step S108) of the presence or absence of liquid in the vicinity of the treated target and the judgment in step S109 are executed as described above. Thus, the judgment parameter η is set to 1 in step S135, only when the phase difference φ(t) between the driving current I and driving voltage V was continuously kept at the

phase threshold φth or more during a predetermined time. Specifically, the judgment section 36 judges that the vicinity of the treated target, with which the end effector 12 is in contact, is not filled with liquid, based on the fact that the phase difference φ(t) was continuously kept at the phase threshold φth or more during the predetermined time (i.e. during a period until the count number M(t) reaches the reference count number Mref from 0).

In the present modification, too, in the state in which the driving current I is being output from the driving circuit (output unit) 22, the above-described constant current control, in which the current value of the driving current I is kept constant (at the reference current value Iref) with the passing of time, is executed. As described in the first embodiment, when the constant current control of the driving current I is executed, the phase difference φ between the driving current I and driving voltage V becomes greater in the state in which the end effector 12 is located in the air (the state in which the vicinity of the treated target is not filled with liquid), than in the state in which the end effector 12 is located in the liquid (the state in which the vicinity of the treated target is filled with liquid). Thus, based on at least whether or not the phase difference φ(t) at time t was the phase threshold φth or more, it is judged whether liquid is filled or not. Thereby, it is properly judged whether the vicinity of the treated target, with which the end effector 12 is in contact, is filled with liquid or not.

Additionally, in the present embodiment, it is judged that the vicinity of the treated target is not filled with liquid, based on the fact that the phase difference φ(t) was continuously kept at the phase threshold φth or more during the predetermined time. Thereby, when the phase difference φ has instantaneously become large due to noise or the like, it is properly judged that the vicinity of the treated target is filled with liquid, and the presence or absence of liquid in the vicinity of the treated target is judged more properly.

In the meantime, in a certain modification, like the modification of FIG. 9, the presence or absence of liquid in the vicinity of the treated target is judged based on the phase difference φ between the driving current I and driving voltage V. However, the process based on the count number M(t) (steps S133, S134 and S138 in FIG. 9) may not be executed. In this case, when the phase difference φ(t) was the phase threshold φth or more, the judgment parameter η is set to 1, and, in step S109 in FIG. 4, the judgment section 36 judges that the vicinity of the treated target, with which the end effector 12 is in contact, is not filled with liquid (step S109-No). On the other hand, when the phase difference φ(t) was less than the phase threshold φth, the judgment parameter η is set to 0, and, in step S109 in FIG. 4, the judgment section 36 judges that the vicinity of the treated target is filled with liquid (step S109-Yes).

Additionally, in a certain modification, instead of judging whether or not the phase difference φ(t) at time t was the phase threshold φth or more, the calculator 35 calculates a variation rate ε(t) which is obtained by subtracting a phase difference φ(t-1) at a time point (t-1), which is a detected object in immediately previous detection prior to time t, from the phase difference φ(t) at the time t. Then, the judgment section 36 judges whether the variation rate ε(t) was positive or not. If the variation rate ε(t) was positive, the judgement section 36 judges that the phase difference φ increased between the time (t-1) and time t. When it was judged that the phase difference φ increased between the time (t-1) and time t, it is judged whether the variation rate (increase rate) ε(t) was a reference increase rate εref or more. When the variation rate (increase rate) ε(t) was the reference increase rate εref or more, the count number M(t) at time t is calculated by adding 1 to the count number M(t-1) at time (t-1).

Also in this modification, like the modification of FIG. 9, when the count number M(t) was the reference count number Mref or more, the judgment section 36 sets the judgment parameter η to 1. Then, in step S109 in FIG. 4, the judgment section 36 judges that the vicinity of the treated target, with which the end effector 12 is in contact, is not filled with liquid (step S109-No). In the meantime, in the present modification, when the variation rate ε(t) was not positive (i.e. 0 or negative) and the variation rate (increase rate) ε(t) was less than the reference increase rate εref, the count number M(t) at time t is reset to 0.

In this modification, by the above-described process being executed, it is judged that the vicinity of the treated target is not filled with liquid (the contact portion of the end effector 12, which is in contact with the treated target, is not located in the liquid), based on the fact that the phase difference φ between the driving current I and driving voltage V continuously increased during a predetermined time at the variation rate ε which is the reference increase rate εref or more.

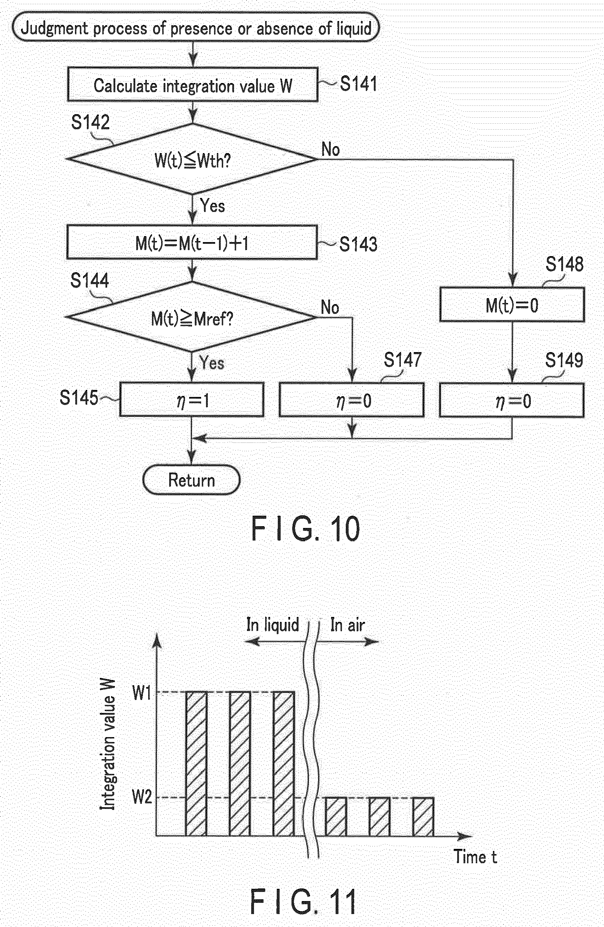

Additionally, in a second modification of the first embodiment, which is illustrated in FIG. 10 and FIG. 11, based on an integration value W of the driving voltage V, it is judged whether the vicinity of the treated target is filled with liquid or not. In the meantime, in the present modification, the process of step S107 in FIG. 4 (the detection of acoustic impedance Z with the passing of time) may not be executed. In addition, in this modification, too, the judgment process (step S108) of the presence or absence of liquid in the vicinity of the treated target is not executed before it is judged in step S105 in FIG. 4 that the end effector 12 is in contact with the treated target.

FIG. 10 is a flowchart illustrating the judgment process (the process of step S108 in FIG. 4) of the presence or absence of liquid in the vicinity of the treated target, the judgment process being executed by the energy control device 3. As illustrated in FIG. 10, in the judgment process of the presence or absence of liquid, the calculator 35 calculates an integration value W of the driving voltage V during a reference time ΔT, based on the detected driving voltage V (step S141). Here, the reference time ΔT is, for example, a half cycle or one cycle of the driving voltage V (driving current I), and the integration value W of the driving voltage V in every half cycle or every cycle of the driving voltage V is calculated.

In this case, for example, an integration value W(t) is calculated, with time t being set as a terminal end of the reference time ΔT. If the integration value W(t) is calculated, the judgment section 36 judges whether or not the calculated integration value W(t) was an integration threshold Wth or less (step S142). Here, the integration threshold Wth may be set by the surgeon or the like, and may be stored in the storage medium 31.

In the present modification, like the modification of FIG. 9, the count number M(t) at time t is specified. In addition, if the integration value W(t) was the integration threshold Wth or less (step S142-Yes), the calculator 35 calculates the count number M(t) at time t, by adding 1 to a count number M(t-1) at a time point (t-1) (step S143). Incidentally, in this modification, too, at the time point when it was judged in step S105 in FIG. 4 that the end effector 12 is put in contact with the treated target, the count number M(t) is set at 0.

In this modification, too, if the count number M(t) is calculated, the judgment section 36 judges whether the calculated count number M(t) was a reference count number Mref or more (step S144). In addition, if the count number M(t) was the reference count number Mref or more (step S144-Yes), the judgment section 36 sets the judgment parameter η to 1 (step S145), and, in step S109 in FIG. 4, the judgment section 36 judges that the vicinity of the treated target, with which the end effector 12 is in contact, is not filled with liquid (step S109-No). On the other hand, if the count number M(t) was less than the reference count number Mref (step S144-No), the judgment section 36 sets the judgment parameter η to 0 (step S147), and, in step S109 in FIG. 4, the judgment section 36 judges that the vicinity of the treated target is filled with liquid (step S109-Yes). In this case, the value, which was obtained by adding 1 to the count number M(t-1) at the time (t-1), is stored as the count number M(t) at time t.

In addition, in step S142, if the integration value W(t) at time t was greater than the integration threshold Wth (step S142-No), the calculator 35 resets the count number M(t) at time t to 0 (step S148). Then, the judgment section 36 sets the judgment parameter η to 0 (step S149), and, in step S109 in FIG. 4, the judgment section 36 judges that the vicinity of the treated target is filled with liquid (step S109-Yes). At this time, the reset value (i.e. 0) is stored as the count number M(t) at time t.

The judgment process (step S108) of the presence or absence of liquid in the vicinity of the treated target and the judgment in step S109 are executed as described above. Thus, the judgment parameter η is set to 1 in step S135, only when the integration value W(t) of the driving voltage V was continuously kept at the integration threshold Wth or less during a predetermined time. Specifically, the judgment section 36 judges that the vicinity of the treated target, with which the end effector 12 is in contact, is not filled with liquid, based on the fact that the integration value W(t) of the driving voltage V was continuously kept at the integration threshold Wth or less during the predetermined time (i.e. during a period until the count number M(t) reaches the reference count number Mref from 0).

FIG. 11 illustrates an example of a variation with time of the integration value W of the driving voltage V in the state in which the end effector 12 located in the liquid is abrading the treated target, and in the state in which the end effector 12 located in the air is abrading the treated target. In FIG. 11, the abscissa indicates time t, with the start of output of the driving current I being set as a reference, and the ordinate indicates the integration value W of the driving voltage V.

In the present modification, too, in the state in which the driving current I is being output from the driving circuit (output unit) 22, the above-described constant current control, in which the current value of the driving current I is kept constant (at the reference current value Iref) with the passing of time, is executed. As described in the first embodiment, when the constant current control of the driving current I is executed, the voltage value of the driving voltage V becomes smaller in the state in which the end effector 12 is located in the air (the state in which the vicinity of the treated target is not filled with liquid), than in the state in which the end effector 12 is located in the liquid (the state in which the vicinity of the treated target is filled with liquid). Thus, as illustrated in FIG. 11, compared to the state in which the end effector 12 is located in the liquid, in the state in which the end effector 12 is located in the air, the integration value W of the driving voltage V during the reference time ΔT (half cycle or one cycle) becomes smaller. In the example illustrated in FIG. 11, in the state in which the end effector 12 located in the liquid is abrading the treated target, the integration value W of the driving voltage V during the half cycle becomes an integration value (first integration value) W1. By contrast, in the state in which the end effector 12 located in the air is abrading the treated target, the integration value W of the driving voltage V during the half cycle becomes an integration value (second integration value) W2 which is less than the integration value W1. Thus, based on at least whether or not the integration value W(t) of the driving voltage V at time t was the integration threshold Wth or less, the presence or absence of liquid in the vicinity of the treated target is judged. Thereby, it is properly judged whether the vicinity of the treated target, with which the end effector 12 is in contact, is filled with liquid or not.

In the meantime, in a certain modification, like the modification of FIG. 10 and FIG. 11, the presence or absence of liquid in the vicinity of the treated target is judged based on the integration value W of the driving voltage V. However, the process based on the count number M(t) (steps S143, S144 and S148 in FIG. 10) may not be executed. In this case, when the integration value W(t) of the driving voltage V was the integration threshold Wth or less, the judgment parameter η is set to 1, and, in step S109 in FIG. 4, the judgment section 36 judges that the vicinity of the treated target, with which the end effector 12 is in contact, is not filled with liquid (step S109-No). On the other hand, when the integration value W(t) was greater than the integration threshold Wth, the judgment parameter η is set to 0, and, in step S109 in FIG. 4, the judgment section 36 judges that the vicinity of the treated target is filled with liquid (step S109-Yes).

(Second Embodiment)

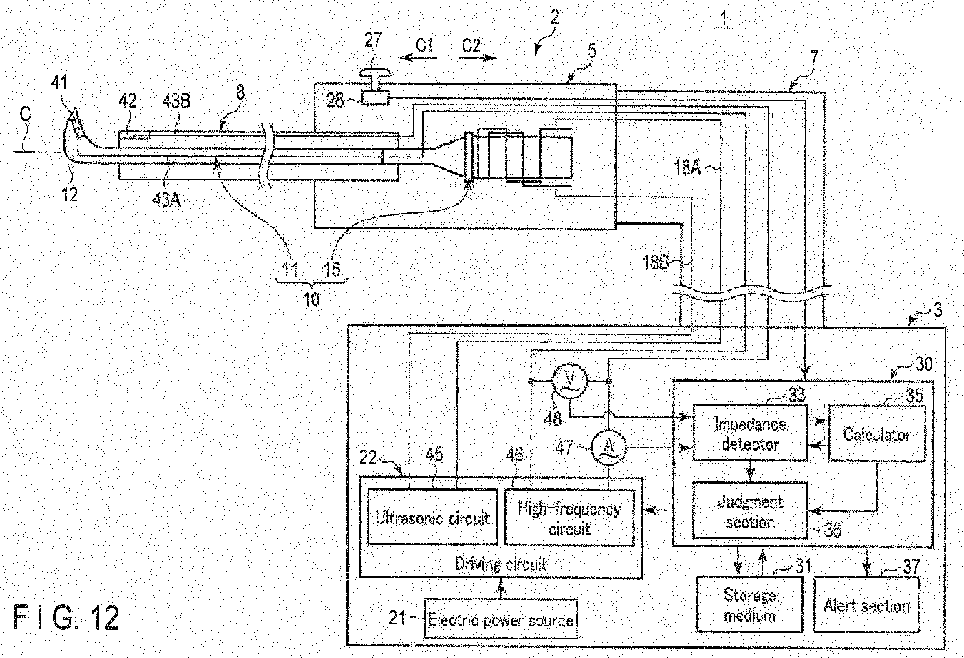

Next, a second embodiment of the present invention will be described with reference to FIG. 12 to FIG. 14. In the meantime, in the second embodiment, the configuration of the first embodiment is modified as described below. Incidentally, the same parts as in the first embodiment are denoted by like reference numerals, and a description thereof is omitted.

FIG. 12 is a view illustrating the configuration of a treatment instrument (energy treatment instrument) 2 and an energy control device 3 of the present embodiment. As illustrated in FIG. 12, in this embodiment, a first electrode 41 is provided on the end effector 12, and a second electrode 42 is provided on a distal portion of the sheath 8. In addition, in this embodiment, the driving circuit (output unit) 22 outputs ultrasonic electric energy (driving current I) and outputs, at the same time, high-frequency electric energy (high-frequency current I') which is different from the ultrasonic electric energy. In the present embodiment, the driving circuit 22 is provided with an ultrasonic circuit 45 which converts electric power from the electric power source 21 to ultrasonic electric energy (AC electric power), and a high-frequency circuit 46 which converts electric power from the electric power source 21 to high-frequency electric energy (high-frequency electric power). By the ultrasonic electric energy (driving current I) being supplied to the vibration generator 15 from the ultrasonic circuit 45, the vibration generator 15 generates ultrasonic vibration. Then, like the first embodiment, the generated ultrasonic vibration is transmitted to the end effector 12 through the vibration transmitting member 11, and the end effector 12 treats a bone or cartilage as a treated target by using the transmitted ultrasonic vibration.

In the present embodiment, the first electrode 41 is electrically connected to the high-frequency circuit 46 of the driving circuit 22 via a high-frequency electric path 43A which extends through the inside of the treatment instrument 2 and the inside of the cable 7. In addition, the second electrode 42 is electrically connected to the high-frequency circuit 46 of the driving circuit 22 via a high-frequency electric path 43B which extends through the inside of the treatment instrument 2 and the inside of the cable 7. Incidentally, the high-frequency electric paths 43A and 43B are electrically insulated. In the present embodiment, ultrasonic electric energy (driving current I) is output from the ultrasonic circuit 45, and, at the same time, high-frequency electric energy is output from the high-frequency circuit 46. The output high-frequency electric energy is supplied to the first electrode 41 through the high-frequency electrical path 43A, and is also supplied to the second electrode 42 through the high-frequency electrical path 43B. Thereby, the first electrode 41 has a first electric potential E1, and the second electrode 42 has a second electric potential E2 that is different from the first electric potential E1. Since the first electrode 41 and second electrode 42 have mutually different electric potentials, a high-frequency voltage V' is applied between the first electrode 41 and second electrode 42. In addition, in the state in which the end effector 12 and the distal portion of the sheath 8 are located in the liquid, a high-frequency current (detection current) I' flows between the first electrode 41 and second electrode 42 through the liquid.

In the present embodiment, there is provided a current detector 47, such as an amperemeter, which detects, with the passing of time, the high-frequency current I' which is output from the driving circuit 22 (high-frequency circuit 46). The current detector 47 is configured to detect the high-frequency current I' flowing in the electrical path (43A or 43B). In addition, in the present embodiment, there is provided a voltage detector 48, such as a voltmeter, which detects, with the passing of time, the high-frequency voltage V' which is applied between the first electrode 41 and second electrode 42 (between the high-frequency electrical paths 43A and 43B) by the output of the high-frequency electric energy from the driving circuit 22 (high-frequency circuit 46). The voltage detector 48 is configured to detect the high-frequency voltage V' (electric potential difference) between the high-frequency electrical paths 43A and 43B.

In this embodiment, the impedance detector 33 detects, with the passing of time, a high-frequency impedance (tissue impedance) Z' of high-frequency electric energy (i.e. between the electrodes 41 and 42), based on the detection results in the current detector 47 and voltage detector 48. If the high-frequency impedance Z' is great, this means a state in which the high-frequency current I' does not easily flow between the first electrode 41 and second electrode 42. On the other hand, if the high-frequency impedance Z' is small, this means a state in which the high-frequency current I' easily flows between the first electrode 41 and second electrode 42. The high-frequency impedance Z' is calculated as indicated by equation (2), based on the high-frequency current (detection current) I' and high-frequency voltage (detection voltage) V'.

$$Z' = V'/I' \qquad\qquad (2)$$

In the present embodiment, the judgment section 36 judges, based on the detection result of the high-frequency impedance Z', whether the vicinity of a treated target (bone or cartilage), with which the end effector 12 is in contact, is filled with a liquid in the joint cavity (70). In addition, like the first embodiment, if the judgment section 36 judged that the vicinity of the treated target is not filled with liquid, the judgment section 36 generates a signal indicating that liquid is not filled. In this embodiment, like the first embodiment, based on a judgment result in the judgment section 36 (as to whether the vicinity of the treated target is filled with liquid or not), the controller 30 controls the output of the driving current I (ultrasonic electric energy) from the driving circuit (output unit) 22, and controls the alert section 37.

Next, the function and the advantageous effects of the present embodiment will be described. In this embodiment, like the first embodiment, in the state in which the liquid L is being supplied to the vicinity of the treated target by the liquid supply assisting instrument 60 or the like, the end effector 12 is put in contact with the treated target, and the treated target is abraded by using ultrasonic vibration. In addition, in this embodiment, high-frequency electric energy is supplied to the first electrode 41 and second electrode 42, at the same time as the ultrasonic vibration is transmitted to the end effector 12. At this time, if the liquid L is properly supplied to the vicinity of the treated target, the vicinity of the treated target is filled with the liquid L, and the end effector 12 and the distal portion of the sheath 8 are located in the liquid L. Thus, if the liquid L is properly supplied to the vicinity of the treated target, the first electrode 41 and second electrode 42 are located in the liquid L. In the meantime, in the state in which the treated target is being abraded by the end effector 12 located in the liquid, the second electrode 42 is not in contact with the treated target. At this time, the first electrode 41 may be in contact with the treated target, or may not be in contact with the treated target.

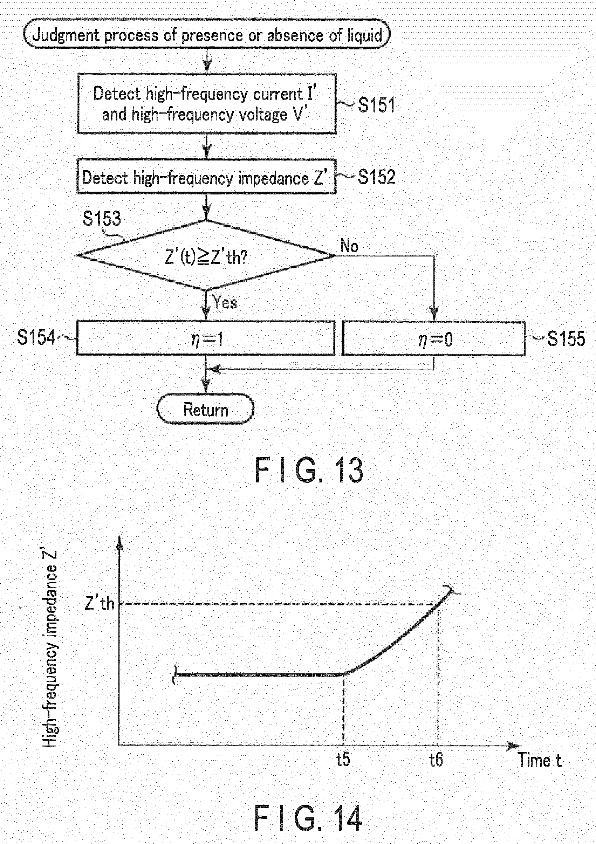

FIG. 13 is a flowchart illustrating a judgment process (the process of step S108 in FIG. 4) of the presence or absence of liquid in the vicinity of a treated target, the judgment process being executed by the energy control device 3 of the present embodiment. Incidentally, in this embodiment, in step S102 in FIG. 4, the output of the driving current I is started, and the output of the high-frequency electric energy (high-frequency current I') is started. In addition, in this embodiment, step S106 and step S107 in FIG. 4 do not need to be executed, and the acoustic impedance Z does not need to be detected after it was judged in step S105 that the end effector 12 came in contact with the treated target (step S105-Yes).

As illustrated in FIG. 13, in the judgment process of the presence or absence of liquid, the current detector 47 continuously detects, with the passing of time, the high-frequency current (detection current) I', and the voltage detector 48 continuously detects, with the passing of time, the high-frequency voltage (detection voltage) V' (step S151). Based on the detected high-frequency current I' and high-frequency voltage (detection voltage) V', the impedance detector 33 continuously detects, with the passing of time, the high-frequency impedance (tissue impedance) Z' (step S152).

If the high-frequency impedance Z' is detected, the judgment section 36 judges whether a high-frequency impedance Z'(t) at time t was an impedance threshold (high-frequency impedance threshold) Z'th or more (step S153). The impedance threshold Z'th may be set by the surgeon or the like, and may be stored in the storage medium 31. If the high-frequency impedance Z'(t) was the impedance threshold (high-frequency impedance threshold) Z'th or more (step S153-Yes), the judgment section 36 sets the judgment parameter η to 1 (step S154). Then, in step S109 in FIG. 4, the judgment section 36 judges that the vicinity of the treated target is not filled with liquid (step S109-No). On the other hand, if the high-frequency impedance Z'(t) was less than the impedance threshold (high-frequency impedance threshold) Z'th (step S153-No), the judgment section 36 sets the judgment parameter η to 0 (step S155). Then, in step S109 in FIG. 4, the judgment section 36 judges that the vicinity of the treated target is filled with liquid (step S109-Yes).

The judgment process (step S108) of the presence or absence of liquid in the vicinity of the treated target and the judgment in step S109 are executed as described above. Thus, the judgment parameter η is set to 1 in step S154, only when the high-frequency impedance Z'(t) has increased to the impedance threshold Z'th or more. Specifically, the judgment section 36 judges that the vicinity of the treated target, with which the end effector 12 is in contact, is not filled with liquid, based on the fact that the high-frequency impedance Z'(t) has increased to the impedance threshold Z'th or more.

Here, in the state in which the vicinity of the treated target is filled with the liquid, the end effector 12 and the distal portion of the sheath 8 are located in the liquid. Thus, the high-frequency current I' easily flows between the first electrode 41 and second electrode 42 through the liquid, and the high-frequency impedance Z' decreases. On the other hand, in the state in which the vicinity of the treated target is not filled with the liquid, the end effector 12 and the distal portion of the sheath 8 are located in the air. Thus, the high-frequency current I' does not easily flow between the first electrode 41 and second 42, and the high-frequency impedance Z' increases.

FIG. 14 is a view illustrating an example of a variation with time of the high-frequency impedance Z'. In FIG. 14, the abscissa indicates time t, with the start of output of the driving current I (high-frequency current I') being set as a reference, and the ordinate indicates the high-frequency impedance Z'. In the example illustrated in FIG. 14, at time t5 or thereabout, there occurs a state in which the vicinity of the treated target, with which the end effector 12 is in contact (i.e. which is being abraded by the end effector 12), is not filled with liquid. Thus, from the time t5 or thereabout, the high-frequency impedance Z' begins to increase with time. In the example illustrated in FIG. 14, in the state in which the high-frequency impedance Z' after the time t5 is increasing with time, the process of steps S108 and S109 in FIG. 4 is repeatedly executed with the passing of time. Then, at time t6, it is judged that the high-frequency impedance Z'(t) has increased to the impedance threshold (high-frequency impedance threshold) Z'th or more, and, at time t6 or immediately thereafter, it is judged by the process of step S109 that the vicinity of the treated target is not filled with liquid (the contact part of the end effector 12, which is in contact with the treated target, is not located in the liquid). Then, the signal indicating that liquid is not filled is generated, and at the time 6 or immediately thereafter, the output of the driving current I is stopped and/or an alert is issued by the alert section 37 by the process of step S112.

In the present embodiment, based on whether or not the high-frequency impedance Z' has increased to the impedance threshold (high-frequency impedance threshold) Z'th or more, it is judged whether the vicinity of the treated target, with which the end effector 12 is in contact, is filled with liquid or not. As described above, in the state in which the vicinity of the treated target is not filled with liquid (i.e. the contact part of the end effector 12, which is in contact with the treated target, is located in the air), the high-frequency impedance Z' increases. Thus, based on whether or not the high-frequency impedance Z'(t) at the time t was the impedance threshold Z'th or more, it is properly judged whether the vicinity of the treated target, with which the end effector 12 is in contact, is filled with liquid or not.

In addition, in the present embodiment, too, based on the signal indicating that the vicinity of the treated target, with which the end effector 12 is in contact, is not filled with liquid, the output of the driving current I is stopped and/or an alert is issued by the alert section 37 by the control of the controller 30. Therefore, in the present embodiment, too, the same function and advantageous effects as in the first embodiment can be obtained.

(Modification of the Second Embodiment)

In the meantime, in a certain modification of the second embodiment, the treatment instrument 2 is not provided with the vibration generator 15, and ultrasonic vibration may not be transmitted to the end effector 12. In this case, the end effector 12 treats the treated target by using only the high-frequency current I' as energy. In the present modification, the driving current (ultrasonic current) I is not output from the driving circuit (output section) 22, and the driving current I is not supplied to the treatment instrument 2. In addition, in this modification, the high-frequency current I' (high-frequency electric energy) is output from the driving circuit 22 as the driving current which drives the treatment instrument 2, and the output high-frequency current I' is supplied to the treatment instrument 2.

In the present modification, like the second embodiment, the first electrode 41 is provided on the end effector 12, and the second electrode 42 is provided on the distal portion of the sheath 8. In addition, if the high-frequency electric energy (high-frequency current I') is output from the driving circuit (output section) 22, the first electrode 41 has the first electric potential E1, and the second electrode 42 has the second electric potential E2 that is different from the first electric potential E1. In this embodiment, too, in the state in which the bone or cartilage is being treated as the treated target in the joint cavity, the vicinity of the treated target is filled with liquid. In addition, in the state in which the end effector 12 is located in the liquid, the end effector 12 is put in contact with the treated target, and the treated target is treated by using the high-frequency current I'. At this time, the first electrode 41 of the end effector 12 is in contact with the treated target, and the second electrode 42 of of the sheath 8 is not in contact with the treated target. Incidentally, in this modification, too, if the liquid L is properly supplied to the vicinity of the treated target in the treatment, the vicinity of the treated target is filled with the liquid L, and the end effector 12 (first electrode 41) and the distal portion (second electrode 42) of the sheath 8 are located in the liquid L.

In the present modification, like the second embodiment, it is judged whether the vicinity of the treated target is filled with liquid or not, based on the high-frequency impedance Z' of the high-frequency electric energy (see FIG. 13). Specifically, if the high-frequency impedance Z'(t) was the impedance threshold (high-frequency impedance threshold) Z'th or more, the judgment section 36 sets the judgment parameter $\eta$ to 1, and, in step S109 in FIG. 4, the judgment section 36 judges that the vicinity of the treated target is not filled with liquid (step S109-No). On the other hand, if the high-frequency impedance Z'(t) was less than the impedance threshold (high-frequency impedance threshold) Z'th, the judgment section 36 sets the judgment parameter $\eta$ to 0, and, in step S109 in FIG. 4, the judgment section 36 judges that the vicinity of the treated target is filled with liquid (step S109-Yes). Accordingly, in this modification, like the second embodiment, based on whether or not the high-frequency impedance Z'(t) at the time t was the impedance threshold Z'th or more, it is properly judged whether the vicinity of the treated target, with which the end effector 12 is in contact, is filled with liquid or not. In the meantime, in the present modification, when it was judged that the vicinity of the treated target is not filled with liquid (step S109-No), the controller 30 stops the output of the high-frequency current (driving current) I', and/or issues an alert by the alert section 37.

(Other Modifications)

In the above-described embodiments, etc., in the energy treatment system (1), the treatment instrument (2) is provided with the end effector (12) configured to treat the treated target by using energy in the state of contact with the treated target in the joint cavity (70), and the driving current (I; I') that drives the treatment instrument (2) is output to the treatment instrument (2) from the output section (22). In the state in which the end effector (12) is in contact with the treated target and the driving current (I; I') is being output from the output section (22), the judgment section (36) is configured to judge whether the vicinity of the treated target, with which the end effector (12) is in contact, is filled with the liquid (L). Then, based on the fact that the judgment section (36) judged that the vicinity of the treated target is not filled with the liquid (L), the controller (30) executes at least either stopping the output of the driving current (I; I') from the output section (22), or issuing an alert to the effect that the liquid (L) is not filled.

If the above-described configurations are satisfied, the above-described embodiments, etc. may be modified as needed, and the above-described embodiments, etc. may be partly combined as needed.

Although the embodiments, etc. of the present invention have been described above, the present invention is not limited to the above-described embodiments, etc., and, needless to say, various modifications can be made without departing from the spirit of the invention.

**Claims**

1.   An energy treatment system comprising:

a treatment instrument including an end effector configured to treat, in a state in which the end effector is in contact with a treated target in a joint cavity, the treated target by using energy;
an output section configured to output to the treatment instrument a driving current which drives the treatment instrument;
a judgment section configured to judge whether a vicinity of the treated target, with which the end effector is in contact, is filled with a liquid, in a state in which the end effector is in contact with the treated target and the driving current is being output from the output section; and
a controller configured to execute at least one of stopping the output of the driving current from the output section and issuing an alert to an effect that the liquid is not filled, based on a fact that the judgment section judged that the vicinity of the treated target is not filled with the liquid.

2.   The energy treatment system of Claim 1, wherein the treatment instrument includes a vibration generator configured to generate ultrasonic vibration, by being supplied with the driving current which was output from the output section, and
the ultrasonic vibration generated by the vibration generator is transmitted to the end effector, and the end effector is configured to treat the treated target by using the transmitted ultrasonic vibration.

3.   The energy treatment system of Claim 2, further comprising a detector configured to detect, with a passing of time, at least one a driving voltage which is applied by the output of the driving current and an acoustic impedance which is calculated based on the driving current and the driving voltage,
wherein the judgment section is configured to judge that the vicinity of the treated target is not filled with the liquid, based on a fact that the acoustic impedance has decreased to an impedance threshold or less, or based on a fact that the driving voltage has decreased to a voltage threshold or less.

4.   The energy treatment system of Claim 3, wherein the controller is configured to cause the output section to output the driving current at a constant current value with the passing of time, thereby causing the end effector to vibrate with a constant amplitude with the passing of time.

5.   The energy treatment system of Claim 2, further comprising:

a detector configured to detect, with a passing of time, the driving current and a driving voltage which is applied by the output of the driving current; and
a calculator configured to calculate a phase difference between the driving current and the driving voltage, based on a detection result of the detector,
wherein the judgment section is configured to judge that the vicinity of the treated target is not filled with the liquid, based on a fact that the phase difference between the driving current and the driving voltage has increased to a phase threshold or more, or based on a fact that the phase difference between the driving current and the driving voltage has continuously increased at an increase rate of a reference increase rate or more during a predetermined time.

6.   The energy treatment system of Claim 2, further comprising:

a detector configured to detect, with a passing of time, a driving voltage which is applied by the output of the driving current; and
a calculator configured to calculate, with the passing of time, an integration value of the driving voltage during a reference time, based on a detection result of the detector,
wherein the judgment section is configured to judge that the vicinity of the treated target is not filled with the liquid, based on a fact that the integration value of the driving voltage has decreased to an integration threshold or less.

7. The energy treatment system of Claim 2, wherein the controller is configured to cause the output section to output the driving current, and to cause the output section to simultaneously output high-frequency electric energy which is different from the driving current,
the treatment instrument includes a first electrode which is provided on the end effector and has a first electric potential by being supplied with the high-frequency electric energy, and a second electrode which has a second electric potential, which is different from the first electric potential, by being supplied with the high-frequency electric energy,
the energy treatment system further includes a detector configured to detect, with a passing of time, a high-frequency impedance of the high-frequency electric energy, and
the judgment section is configured to judge that the vicinity of the treated target is not filled with the liquid, based on a fact that the high-frequency impedance has increased to an impedance threshold or more.

8. An energy control device which is used together with a treatment instrument including an end effector configured to treat, in a state in which the end effector is in contact with a treated target in a joint cavity, the treated target by using energy, the energy control device being configured to control supply to the treatment instrument of a driving current which drives the treatment instrument, the energy control device comprising:

an output section configured to output the driving current to the treatment instrument;
a judgment section configured to judge whether a vicinity of the treated target, with which the end effector is in contact, is filled with a liquid, in a state in which the end effector is in contact with the treated target and the driving current is being output from the output unit; and
a controller configured to execute at least one of stopping the output of the driving current from the output section and issuing an alert to an effect that the liquid is not filled, based on a fact that the judgment section judged that the vicinity of the treated target is not filled with the liquid.

F I G . 1

F I G. 2

EP 3 199 113 A1

F I G. 3

FIG. 4

Judgment process of presence or absence of liquid

S121  Z(t)≦Zth2?  No

Yes

S122  $\eta = 1$

S123  $\eta = 0$

Return

# FIG. 5

Acoustic impedance Z

Zth1

Zth2

t1  t2  t3  t4  Time t

# FIG. 6

FIG. 7

| Conditions | Voltage V (Peak-to-peak value Vpp) | Phase difference $\phi$ | Acoustic impedance Z |
|---|---|---|---|
| In air | 488V | 45.9° | 487.9Ω |
| | 552V | 53.0° | 488.5Ω |
| In liquid | 1170V | 15.1° | 1569.2Ω |
| | 1260V | 24.3° | 1630.7Ω |

FIG. 8

Judgment process of presence or absence of liquid

Calculate phase difference $\phi$ ~S131

S132

$\phi(t) \geqq \phi th?$ — No

Yes

M(t)=M(t−1)+1 ~S133

S138

M(t)=0

S134

M(t)≧Mref? — No

Yes

S135~ $\eta = 1$ | $\eta = 0$ S137 | $\eta = 0$ S139

Return

FIG. 9

Judgment process of presence or absence of liquid

Calculate integration value W — S141

S142

W(t)≦Wth? — No

Yes

$M(t)=M(t-1)+1$ — S143

S144

$M(t)≧Mref?$ — No

S148

$M(t)=0$

Yes

S145 — $\eta=1$

S147 — $\eta=0$

S149 — $\eta=0$

Return

FIG. 10

In liquid | In air

Integration value W

W1

W2

Time t

FIG. 11

25

F I G. 12

Judgment process of presence or absence of liquid

Detect high-frequency current I'
and high-frequency voltage V'    ~S151

Detect high-frequency impedance Z'    ~S152

S153

$Z'(t) \geqq Z'th$?    No

Yes

S154~ $\eta = 1$    $\eta = 0$    ~S155

Return

F I G. 13

Z'th

High-frequency impedance Z'

t5    t6    Time t

F I G. 14

EP 3 199 113 A1

# INTERNATIONAL SEARCH REPORT

| | International application No. |
|---|---|
| | PCT/JP2016/070307 |

### A. CLASSIFICATION OF SUBJECT MATTER
*A61B17/32*(2006.01)i, *A61B17/56*(2006.01)i, *A61B18/12*(2006.01)i

According to International Patent Classification (IPC) or to both national classification and IPC

### B. FIELDS SEARCHED

Minimum documentation searched (classification system followed by classification symbols)
A61B17/32, A61B17/56, A61B18/12

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | US 2013/0282038 A1 (DANNAHER, William D.), 24 October 2013 (24.10.2013), paragraphs [0030] to [0048]; fig. 1 to 4 & JP 2015-515344 A & WO 2013/158545 A1 & CA 2870536 A1 | 1–8 |
| A | JP 2008-55151 A (Olympus Medical Systems Corp.), 13 March 2008 (13.03.2008), paragraphs [0064] to [0082]; fig. 5 & US 2008/0058803 A1 paragraphs [0088] to [0106]; fig. 5 & EP 1894532 A1 | 1–8 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 July 2016 (29.07.16) | 09 August 2016 (09.08.16) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

International application No.

PCT/JP2016/070307

C (Continuation). DOCUMENTS CONSIDERED TO BE RELEVANT

| Category* | Citation of document, with indication, where appropriate, of the relevant passages | Relevant to claim No. |
|---|---|---|
| A | JP 2009-247893 A (Olympus Medical Systems Corp.), 29 October 2009 (29.10.2009), entire text; all drawings & US 2009/0248002 A1 entire text; all drawings & EP 2106762 A1 | 1-8 |
| A | JP 2000-506405 A (Gyrus Medical Ltd.), 30 May 2000 (30.05.2000), entire text; all drawings & US 6015406 A entire text; all drawings & GB 2308980 A & WO 1997/024994 A1 | 1-8 |
| P,A | WO 2015/122307 A1 (Olympus Corp.), 20 August 2015 (20.08.2015), entire text; all drawings (Family: none) | 1-8 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010087060 A